(19) Europäisches Patentamt
European Patent Office
Office européen des brevets

(11) EP 0 606 103 B1

(12) **EUROPEAN PATENT SPECIFICATION**

(45) Date of publication and mention of the grant of the patent:
**12.03.2003 Bulletin 2003/11**

(21) Application number: **94101416.9**

(22) Date of filing: **26.10.1989**

(51) Int Cl.[7]: **C07D 495/22**, C07D 495/14, A61K 31/55
// (C07D495/22, 333:00, 249:00, 243:00, 221:00), (C07D495/22, 333:00, 249:00, 243:00, 209:00), (C07D495/14, 333:00, 249:00, 243:00)

(54) **Thieno 3.2-f 1,2,4 triazolo 4,3-a 1,4 -diazepine derivatives as PAF antagonists**

Thieno 3,2-f 1,2,4 triazolo 4,3-a 1,4 diazepinderivate als PAF-Antagonisten

Dérivés de thieno 3,2-f 1,2,4 triazolo 4,3-a 1,4 diazépine comme antagonistes du PAF

(84) Designated Contracting States:
**AT BE CH DE ES FR GB GR IT LI LU NL SE**

(30) Priority: **31.10.1988 JP 27546088**
**24.11.1988 JP 29706888**
**16.12.1988 JP 31801688**
**28.12.1988 JP 33162288**

(43) Date of publication of application:
**13.07.1994 Bulletin 1994/28**

(62) Document number(s) of the earlier application(s) in accordance with Art. 76 EPC:
**89119910.1 / 0 367 110**

(73) Proprietor: **Eisai Co., Ltd.**
**Tokyo (JP)**

(72) Inventors:
- **Okano, Kazuo**
  **Yawaharamura, Tsukuba-gun, Ibaraki (JP)**
- **Miyazawa, Shuhei**
  **Tsukuba-shi Ibaraki (JP)**
- **Clark, Richard Stephen John**
  **Tsukuba-shi, Ibaraki (JP)**
- **Abe, Shinya**
  **Ushiku-shi, Ibaraki (JP)**
- **Kawahara, Tetsuya**
  **Tsukuba-shi, Ibaraki (JP)**
- **Shimomura, Naoyuki**
  **302, Matsushiro Parkside height**
  **Tsukuba-shi, Ibaraki (JP)**
- **Asano, Osamu**
  **Tsukuba-shi, Ibaraki (JP)**
- **Yoshimura, Hiroyuki**
  **Tsukuba-shi Ibaraki (JP)**
- **Miyamoto, Mitsuaki**
  **Ibaraki (JP)**
- **Sakuma, Yoshinori**
  **Tsuchiura-shi, Ibaraki (JP)**
- **Muramoto, Kenzo**
  **Tsukuba-shi, Ibaraki (JP)**
- **Obaishi, Hiroshi**
  **Tsukuba-shi, Ibaraki (JP)**
- **Harada, Koukichi**
  **Tsukuba-shi, Ibaraki (JP)**
- **Tsunoda, Hajime**
  **Tsukuba-shi, Ibaraki (JP)**
- **Katayama, Satoshi**
  **Tsukuba-shi, Ibaraki (JP)**
- **Yamada, Kouji**
  **Ushiku-shi, Ibaraki (JP)**
- **Souda, Shigeru**
  **Ushiku-shi, Ibaraki (JP)**
- **Machida, Yoshimasa**
  **Tsukuba-shi, Ibaraki (JP)**
- **Katayama, Kouichi**
  **Tsukuba-shi, Ibaraki (JP)**
- **Yamatsu, Isao**
  **Ushiku-shi, Ibaraki (JP)**

(74) Representative:
**Hansen, Bernd, Dr. Dipl.-Chem. et al**
**Hoffmann Eitle,**
**Patent- und Rechtsanwälte,**
**Arabellastrasse 4**
**81925 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

**(Cont. next page)**

(56) References cited:
**EP-A- 0 194 416**
**EP-A- 0 230 942**
**DE-A- 3 724 031**

Remarks:
The file contains technical information submitted after the application was filed and not included in this specification

## Description

The invention provides new 1,4-diazepine derivatives and a pharmacologically acceptable salt thereof and their pharmaceutical use. The compound and the salt have an excellent medical activity.

Prior Art

In recent years, a platelet activating factor (hereinafter referred to simply as PAF) has attracted much attention and its relationship with various diseases is now being clarified. Now, it has been assumed that PAF takes part in not only inflammation, but also DIC, endotoxin shock, asthma, ulcers in alimentary canal, hepatisis and the rejection at the time of organ transplantation. In addition, attention has been drawn to as a mediator which is one of allergic reactions.

Under these circumstances, there have been made investigations on compounds having the anti-PAF activity. Among these compounds, a 1,4-diazepine compound having the anti-PAF action has been proposed, for example, in Japanese Laid-open Patent Application No. 63-33382. However, a satisfactory anti-PAF agent which is adapted, particularly, for allergies such as asthma has not been developed yet.

Accordingly; we have continued investigations and studies over a long term with respect to 1,4-diazepine derivatives which have not only an excellent PAF-inhibiting activity, but also a long activity.

DE-A-37 24 031 reveals cetracine compounds showing the following formulae Ia and Ib

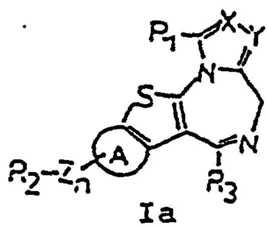

Ia

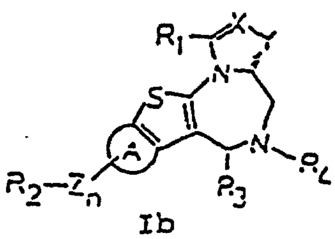

Ib

These compounds are said to have PAF antagonist activity. Specific embodiments thereof involve 3-thioacetyl-6-(2-chlorophenyl)-11-methyl-2,3,4,5,-tetrahydro-8H-pyrido[4',3':4,5]thieno[3,2-f][1,2,4]-triazolo[4,3-a][1,4]diazepine (example 25), 6-(2 chlorophenyl)-11-methyl-2,3,4,5-tetrahydro-8-H-pyrido-[4',3':4,5]thieno[3,2-f][1,2,4]triazolo[4,3-a][1,4]-diazepine, (example 26), and 3-(carboethoxymethyl)-6-(2-chlorophenyl)-11-methyl-2,3,4,5-tetrahydro-8-H-pyrido[4',3':4,5]thieno[3,2-f][1,2,4]-triazolo[4,3-a][1,4]-diazepine (example 27). Example 25 has an anti-PAF activity of $10^{-6}$ mol ($IC_{50}$).

EP-A-0 194 416 discloses thieno-triazolo-1-4-diazepino-2-amides with the following formula I

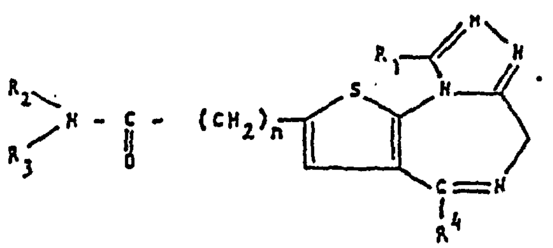

(I)

EP-A-0 230 942 discloses thieno-1,4-diazepine compounds of the general formula

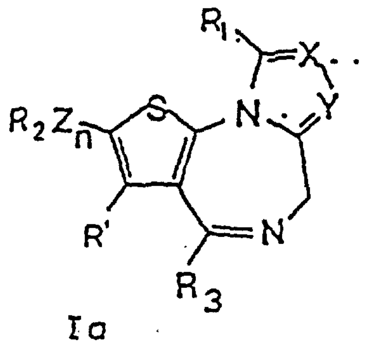

Ia

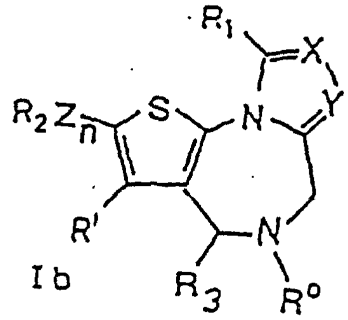

Ib

The above compounds are also used for the treatment of diseases connected with PAF. EP-A-0 268 242 A1 discloses a thienotriazolodiazepine compound of the general formula:

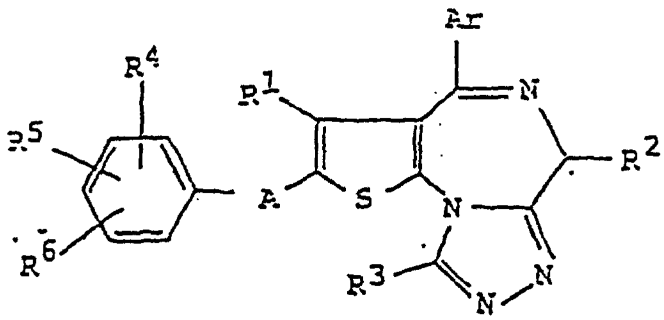

(I)

Said compounds exhibit PAF-antagonistic activity and are useful for the prevention or treatment of various PAF-induced diseases.

A solution to the above defined problem is provided by the present invention directed to certain triazolo-1,4-diazepine compounds of one of the formulae given below or a pharmacologically acceptable salt thereof

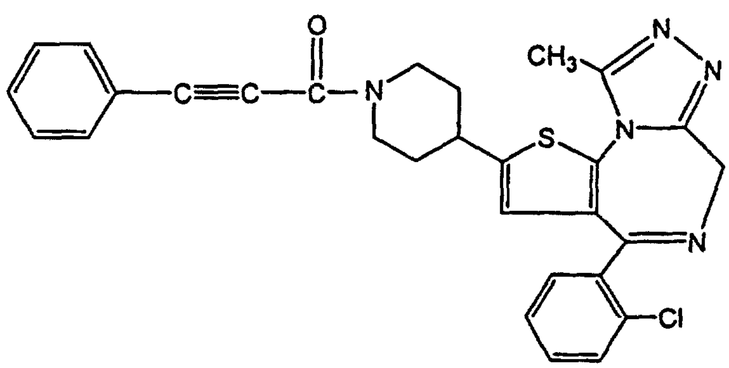

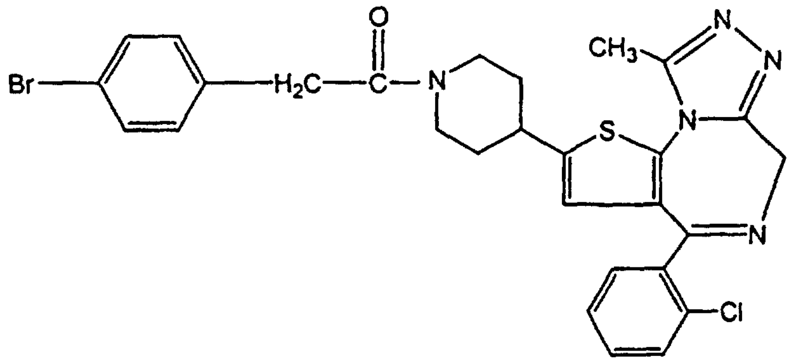

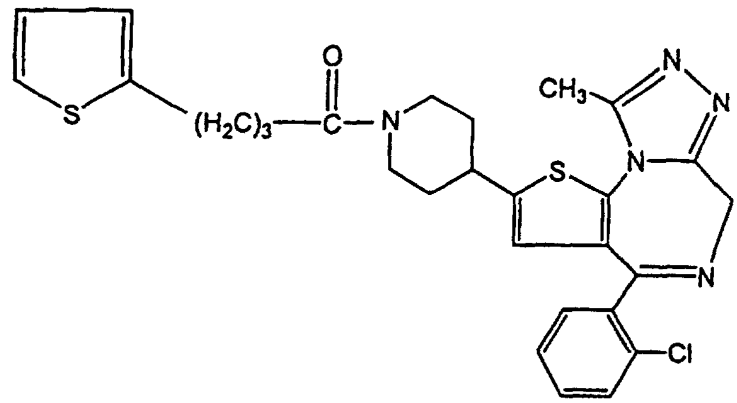

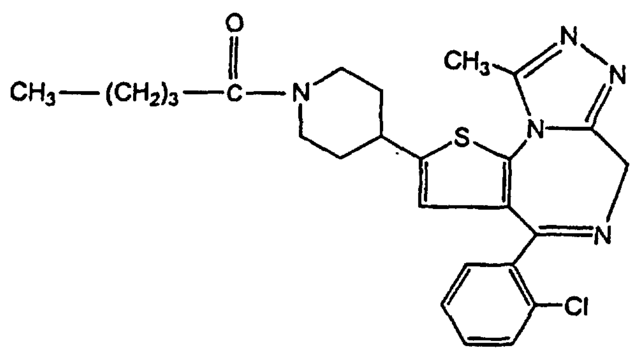

The present invention further relates to a pharmaceutical composition which comprises a pharmacologically effective amount of a compound or a salt thereof, as defined above and a pharmacologically acceptable carrier.

The invention also provides the pharmacological use of the compound as defined above and its salt. In the invention a pharmaceutical composition comprises a pharmacologically effective amount of the compound or the salt thereof, defined above, and a pharmacologically acceptable carrier. A method for treating a disease against which anti-PAF activity is effective, which comprises administering a pharmacologically effective amount of the compound or the salt thereof as defined above. The disease is an allergic disease such as athma.

The 1,4-diazepine derivatives of the invention have good PAF-inhibiting efficacy and good persistency with high safety.

Accordingly, an object of the invention is to provide novel 1,4-diazepine derivatives or pharmacologically acceptable salts thereof which have good anti-PAF action. Another object of the invention is to provide a process for preparing the same. A further object pf the invention is to provide an agent comprising the same.

The pharmacologically acceptable salts used in the present invention are ordinarily employed innoxious salts such as, for example, inorganic salts such as hydrochlorides, hydrobromides, sulfates, phosphates, organic salts such as acetates, maleats, succinates, methanesulfonates, and salts of amino acids such as alginine, aspartic acid, glutamic

acid.

Moreover, some compounds may form hydrates, which are also within the scope of the invention.

The compounds of the invention are prepared by usual procedures. The following illustrates a synthesis technique which can be used for preparing the claimed compounds.

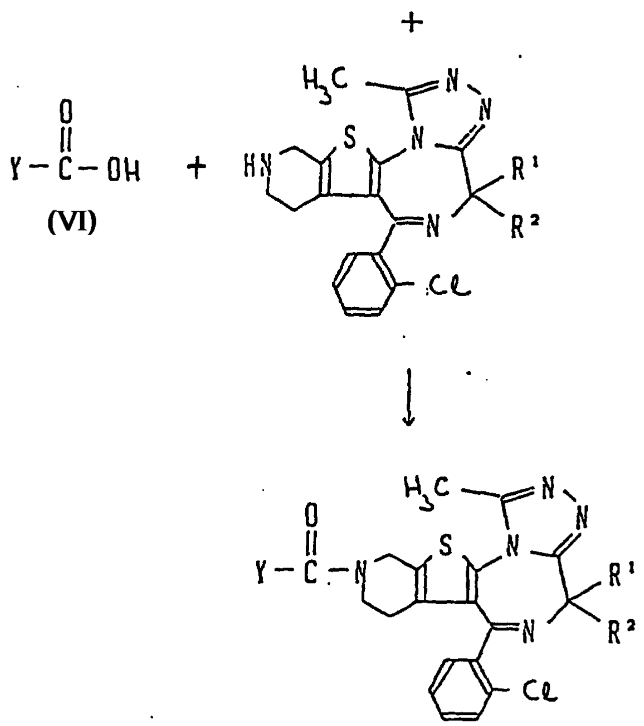

(VI) (III)

$R^1, R^2 = (H, CH_3)$

(I"), not within the scope of the present invention

The carboxylic acid of the general formula (VI) or its reactive derivative and the compound of the general formula (III) are subjected to condensation reaction to obtain the compound of the general formula (I") which shares structural features with the claimed compounds.

This condensation reaction is carried out by usual manner. The reactive derivatives include: acid halides such as acid chlorides, acid bromides; acid azides; N-hydroxybenzotriazole; active esters such as N-hydroxysuccinimide; symmetric acid anhydrides; mixed acid anhydrides with alkali carbonates, p-toluenesulfonic acid.

This reaction is carried out by heating in a solvent-free condition or in a solvent not taking part in the reaction, e.g. benzene, toluene, xylene, tetrahydrofuran, chloroform, carbon tetrachloride, dimethylformamide, thereby causing, for example, dehalogenation reaction. Better results are obtained when the reaction is effected in the presence of inorganic salts such as sodium hydrogencarbonate, potassium carbonate, sodium carbonate, caustic soda or organic bases such as triethylamine, pyridine, pyrimidine, diethylaniline.

When free carboxylic acids are used, better results are obtained for the reaction in the presence of a condensing agent such as dicyclohexylcarbodiimide or 1,1'-carbonyldiimidazole.

The starting compound (III) used in the above Preparation Process can be prepared, for example, according to the following procedure.

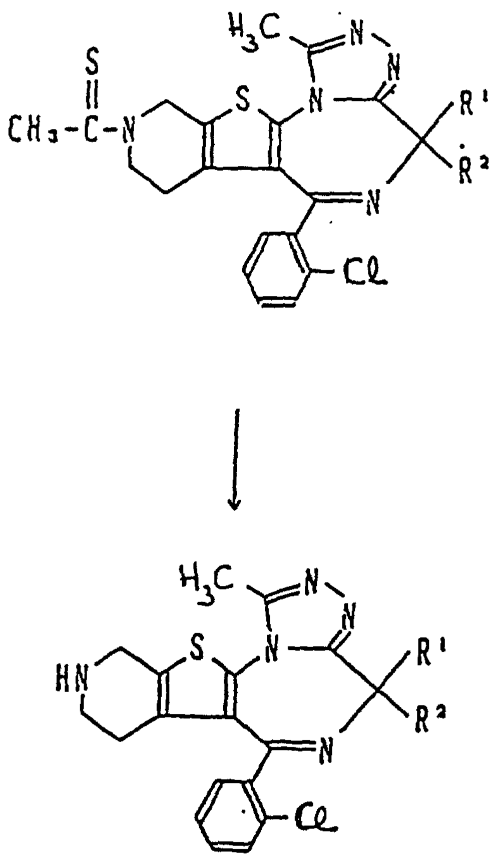

(IX)

(III) ,

wherein $R^1$ and $R^2$ are hydrogen or methyl.

In the above reaction, the thioamide compound of the general formula (IX) is subjected to hydrolysis reaction to obtain the compound of the general formula (III).

The present reaction is carried out by usual manner wherein the compound of the general formula (III) can be obtained by heating in the presence, for example, of sodium hydroxide, potassium hydroxide, sodium ethoxide, sodium methoxide, potassium ethoxide or potassium methoxide. For the reaction, there may be used a solvent such as, for example, an alcohol solvent such as methyl alcohol or ethyl alcohol, tetrahydrofuran, dimethoxyethane, or a hydrous solvent.

The effects of the invention are more particularly described by way of experimental example.

Experimental Example

PAF Recepteor Binding Assay to the Human Platelet

(Method)

Platelets are obtained from healthy men according to usual method and suspended at a concentration of $10^8$ platelets/460 μl in a binding buffer (10 mM phosphate-buffers saline (pH 7.0), with 0.1% (w/V) BSA and 0.9 mM $CaCl_2$). Platelets ($10^8$) in 460 μl of the buffer were added to polypropylene tubes and preincubated with test compounds (20 μl), after vortexing, for 6 min at 37 °C. Subsequently, 20 μl of a binding buffer solution of $^3$H-PAF (final $^3$H-PAF concentration 0.6 - 1 nM) was added to the tubes, which were incubated for 6 minutes. The binding reaction was stopped by adding of 3 ml of an ice-cold washing solution (saline containing 0.1% (w/v) BSA). Platelets were isolated by vaccum filtration on glass filters (Whatman GF/C). After drying the glass filter, radioactivity on the glass filter was measured in scintilator with a liquid scintillation counter.

The inhibition percent is caluculated according to the following equation and the value of IC is determined by interpolation from the figure.

Inhibition % =

$$\frac{\text{(total binding) - (total binding with compound)}}{\text{(total binding ) - (non-specific binding total}}$$

binding : radioactivity of binding in the absence of cold PAF or test compounds
non-specific binding : radioactivity of binding in the presence of $10^{-5}$ M PAF
non-specific binding : radioactivity (dpm) after the incubation with $10^{-5}$ M of cold PAF.

The invented compounds have anti-PAF activity. Moreover, it has been found that the compounds possess more potent and long-acting anti-PAF activity, and show better safety properties than known compounds. Thus, the present invention has a great merit.

Accordingly, the compounds will be effective for the therapy and prophylaxis of all diseases mediated by PAF.

Typical diseases for which the compounds are useful as a therapeutic and prophylactic agent include allergic diseases, asthma, thrombosis, cerebral apoplexy (cerebral hemorrhage, cerebral thrombosis), myocardial infarction, (angina pectoris), human disseminated intravascular coagulation syndrome (DIC), thrombophlebitis, glomerular hepatitis, anaphylactic shock, hemorrhagic shock and the like. The invented compounds will be particularly useful as an anti-allergic agent and an anti-asthmatic agent.

When these compounds are administered as an anti-PAF agent, they may be useful to orally dose in the form of a tablet, powder, granule, capsule, syrup or the like. Alternatively, they may be parenterally dosed as a suppositoty, injection, external remedy or drip. In the case of the invention, the compounds should preferably be used as an oral agent.

The dosage may depend on the type of disease, the degree of symptom and the age. When these compounds are orally administered, the doses of 0.001 - 10 mg/kg, preferably 0.01 - 0.5 mg/kg, will be benefitial.

For the preparations to use as peroral and parenteral dose, they are made using ordinary, pharmaceutically acceptable additives. For the preparation of injections or drips, pH modifiers, buffer solutions, stabilizers and solubilizers are added to the principal ingredient, if necessary. The mixture can be freeze-dried, if necessary, to made injections for subcutaneous, intramuscular or intervenous or drip administrations.

Examples

The starting compounds or substances are prepared according to the follows Preparatory Examples.

Preparatory Example 1

1-Benzyloxycarbonyl-4-(2-hydroxyethyl)piperidine

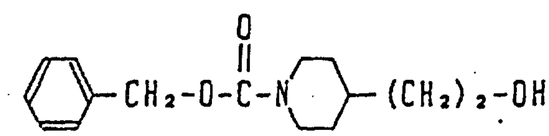

50 g of 4-piperidine ethanol and 49.2 g of sodium hydrogencarbonate were dissolved in 480 ml of water, in which 55.2 ml of benzyloxycarbonyl chloride was gradually dropped under ice-cooling conditions, followed by agitation for 1 hour as it is. The reaction solution was extracted with chloroform and dried with anhydrous magnesium sulfate. The sulfate was filtered off and the solvent was distilled off, after which the resultant residue was subjected to silica gel column chromatography (developing solvent: ethyl acetate:hexane=30:70) to obtain 66.0 g of the captioned compound (yield: 65%).

$^1$H-NMR($CDCl_3$)δ:
0.75-1.85(m,7H), 2.5-3.0(m,2H), 3.4-3.8(m.2H), 3.9-4.3(m,2H). 5.11(s,2H), 7.1-7.4(m.5H)

Preparatory Example 2

1-Benzyloxycarbonyl-4-(formylmethyl)piperidine

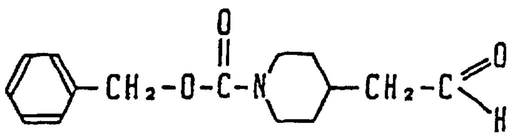

159 g of oxalic acid chloride was dissolved in one liter of dichloromethane, in to which 195.8 g of dimethyl sulfoxide was dropped at -67°C, followed by agitation for 30 minutes. Thereafter, 200 ml of dichloromethane dissolving 66 g of 1-benzyloxycarbonyl-4-(2-hydroxyethyl)-piperidine was dropped at -67°C. Subsequently, 380 g of triethylamine was dropped at -67°C, followed by agitation for about 1 hour. After removal of the solvent by distillation, ethyl acetate was added and insoluble matters were removed by filtration, and the resultant filtrate was washed with water and dried with anhydrous magnesium sulfate, followed by filtration and removal of the solvent by distillation. The resultant residue was subjected to silica gel column chromatography (developing solvent: ethyl acetate:hexane=20:80) to obtain 55.0 g of the captioned compound (yield 84%).

$^1$H-NMR(90 MHz, $CDCl_3$)δ:

0.8-1.85(m,4H), 1.85-2.45(m,1H), 2.36(dd,J=6.1Hz, 1.8Hz,2H), 2.5-3.0(m.2H), 3.9-4.35(m,2H), 5.07(s.2H), 7.1-7.5(m.5H), 9.67(t,J=1.8Hz,1H)

Preparatory Example 3

2-Amino-5-[4-(1-benzyloxycarbonyl)piperidyl]-3-(2-chlorobenzoyl)thiophene

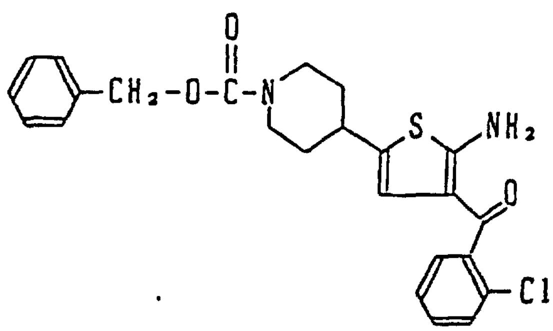

55.0 g of 1-benzyloxycarbonyl-4-(formylmethyl)-piperidine, 6.75 g of sulfur and 38.87 g of 2-chlorocyanoacetophenone were suspended in 250 ml of N,N-dimethylformamide, to which 7.75 g of triethylamine was added at 40°C, followed by agitation for 1.5 hours. After removal of the solvent by distillation, ethyl acetate was added, followed by washing with water and then with a saturated saline solution and drying with anhydrous magnesium sulfate. The reaction solution was filtered and after removal of the solvent by distillation, the resultant residue was subjected to silica gel column chromatography (developing solvent: ethyl acetate:hexane=30:70) to obtain 67.9 g of the captioned compound (yield: 76%).

$^1$H-NMR(90 MHz, $CDCl_3$)δ:

1.15-2.1(m,4H), 3.4-4.05(m,3H), 3.95-4.35(m,2H), 5.06(s,2H), 6.04(bs,1H), 6.94(bs,2H), 7.1-7.55(m,9H)

Preparatory Example 4

2-[4-(1-benzyloxycarbonyl)piperidyl]-5-(bromoacetylamino)-4-(2-chlorobenzoyl)thiophene

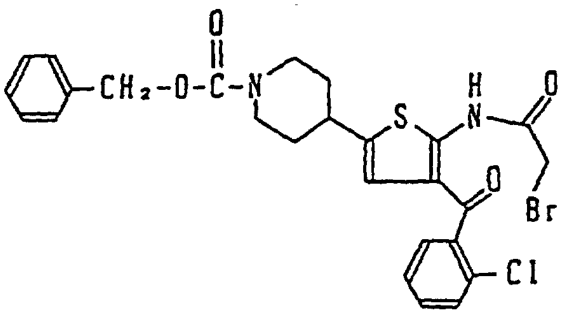

1.5 liters of toluene, 350 ml of water and 27 g of sodium hydrogencarbonate were added to 67.9 g of the compound obtained in Preparatory Example 3, to which was further added 48.61 g of bromoacetic acid bromide at 60°C. After completion of the reaction, ethyl acetate was added, and the resultant organic phase was collected, washed with a saturated saline solution and dried with anhydrous magnesium sulfate. The solution was filtered and the solvent was distilled off to obtain the captioned compound.

$^1$H-NMR(90 MHz, $CDCl_3$)δ:

1.1-2.2(m,4H), 2.55-3.05(m,3H), 4.07(s,2H), 4.0-4.45(m,2H), 5.06(s,2H), 6.36(bs,1H), 7.1-7.6(m,9H) 12.47(bs, 1H)

Preparatory Example 5

2-(Amlnoacetylamino)-5-[4-(1-benzyloxycarbonyl)-piperidyl]-3-(2-chlorobenzoyl)thiophene

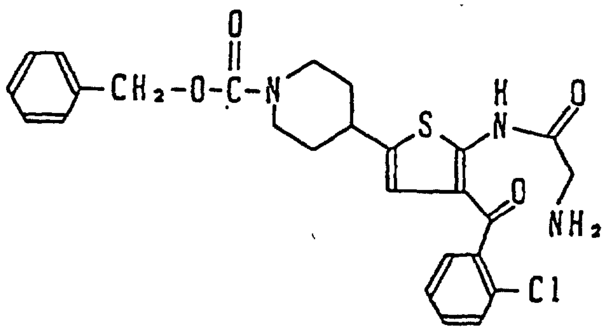

2 liters of ethyl acetate was added to the whole amount of the compound obtained in Preparatory Example 4, after which ammonia gas was passed into the mixture while agitating at room temperature for 3 hours, followed by continuing the agitation for further 12 hours. After passage of nitrogen gas for about 30 minutes, an organic phase was collected under salting-out and the resultant aqueous phase was extracted with chloroform under salting-out.' Both organic phases were dried with anhydrous magnesium sulfate. The solution was filtered and the solvent was distilled off to obtain the captioned compound.

$^1$H-NMR(90 MHz, $CDCl_3$)δ:

1.1-2.15(m,4H), 2.5-3.05(m,3H), 4.61(bs,2H), 3.9-4.4(m.2H), 5.06(s,2H), 6.32(bs,1H), 7.1-7.6(m.9H)

Preparatory Example 6

2-[4-(1-benzyloxycarbonyl)piperidyl]-4-(2-chlorophenyl)-thieno[3,2-f][1,4]diazepin-7-one

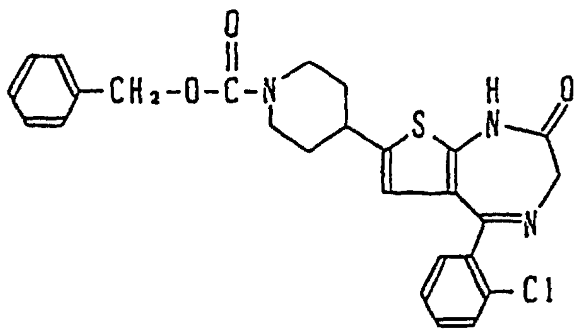

600 ml of benzene, 1.5 liters of pyridine and 9.6 g of acetic acid were added to the whole amount of the compound obtained in Preparatory Example 5, followed by refluxing for 25 hours while removing the water to outside. After removal of the solvent by distillation, the resultant residue was subjected to silica gel column chromatography (developing solvent: ethyl acetate:hexane = 60:40) to obtain 61.21 g of the captioned compound (total yield of the three steps: 77%).

$^1$H-NMR(90 MHz, $CDCl_3$)δ:

1.0-2.15(m,4H), 2.35-3.0(m,3H), 3.9-4.4(m,2H), 4.43(m,2H), 5.07(s,2H), 6.16(bs,1H), 7.05-7.5S(m. 9H), 8.93 (bs,1H)

Preparatory Example 7

2-[4-(1-benzyloxycarbonyl)piperidyl]-4-(2-chlorophenyl)-thieno[3,2-f][1,4]diazepin-7-thione

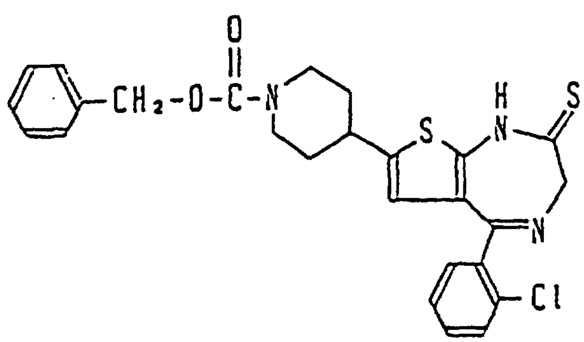

61.21 g of the compound obtained in Preparatory Example 6, 13.53 of sodium hydrogencarbonate and 27.54 g of phosphorus pentasulfide were suspended in 1 liter of 1,2-dimethoxyethane, and agitated at 80°C for 1.5 hours. The reaction solution was filtered through the Celite membrane and the resultant filter cake was washed with chloroform and methanol (Ca 7:3). The washing was combined with the filtrate. The solvent was distilled off and the residue was subjected to silica gel column chromatography (developing solvent: dichloromethane containing 2 - 8% of methanol) to obtain 62.6 g of the captioned compound (yield 99%).

$^1$H-NMR(90 MHz, $CDCl_3$)δ:

1.15-2.1(m,4H), 2.5-3.1(m,3H), 3.95-4.45(m,2H). 4.83(bs,2H), 5.07(bs,2H), 6.18(bs,1H), 7.0-7.6(m,9H)

Preparatory Example 8

2-[4-(1-benzyloxycarbonyl)piperidyl]-4-(2-chlorophenyl)-9-methyl-6H-thieno[3,2-f][1,2,4]-triazolo[4,3-a][1,4]diazepine

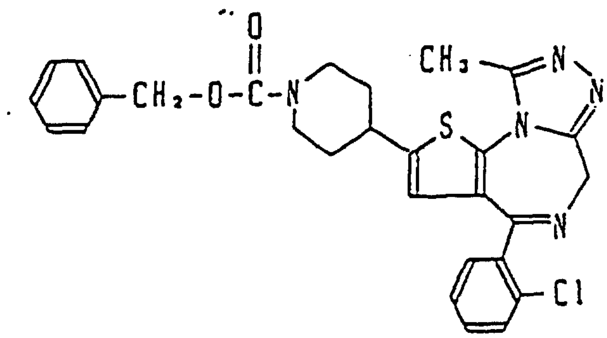

62.6 g of the compound obtained in Preparatory Example 7 was suspended in 3.5 liters of methanol, to which 33.5 g of hydrazine monohydrate was added, followed by agitation at room temperature for 1 hour. After removal of the solvent by distillation, and agitated at 80°C for 1 hour. After removal of the solvent by distillation, the resultant reside was subjected to silica gel column chromatography (developing solvent: dichloromethane:methanol=98:2) to obtain 21.5 g of the captioned compound (yield 33%).

$^1$H-NMR(90 MHz, $CDCl_3$)δ:

1.2-2.2(m,4H), 2.6-3.05(m,3H), 2.67(s,2H), 4.0-4.4(m,2H), 4.88(bs,2H), 5.07(bs,2H), 6.34(bs,1H), 7.1-7.5(m,9H)

Preparatory Example 9

4-(2-Chlorophenyl)-9-methyl-2-(4-piperidyl)-6H-thieno[3,2-f][1,2,4]triazolo[4,3-a][1,4]diazepine

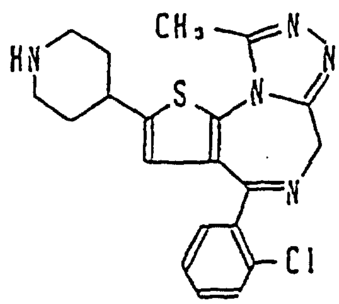

6.65 g of the compound obtained in Preparatory Example 8 was dissolved in 140 ml of dichloromethane, to which 17 ml of trimethylsilyl iodide was added in a stream of nitrogen, followed by agitation in nitrogen for 25 minutes. After cooling, 40 ml of methanol was added and the solvent was distilled off. The resultant residue was subjected to silica gel column chromatography (developing solvent: dichloromethane:methanol:triethylamine=94.5:5:0.5) to obtain 4.45 g of the captioned compound (yield 90%).

$^1$H-NMR(90 MHz, $CDCl_3$)δ:

1.1-2.05(m,4H), 2.35-3.2(m,5H), 2.60(s,3H), 4.77(bs, 2H), 6.37(bs,1H), 7.2-7.6(m,4H)

MS m/z (Pos. FAB): 398(M+H)$^+$

Example 1

4-(2-Chlorophenyl)-9-methyl-2-[4-(1-phenylpropiolylpiperidyl)]-6H-thieno[3,2-f][1,2,4]-triazolo[4,3-a][1,4]diazepine

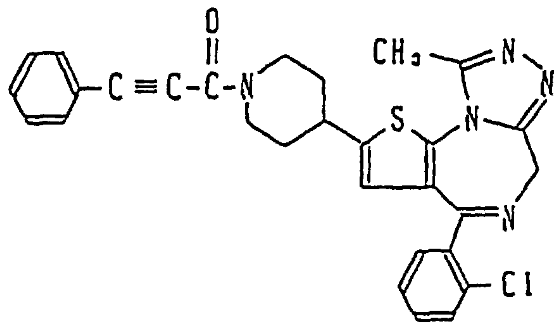

50 mg of phenylpropiolic acid, 110 mg of the piperidine product obtained in Preparatory Example 9 and 50 mg of 1-hydroxybenzotriazole monohydrate were dissolved in 8 ml of N,N-dimethylformamide, to which 70 mg of N, N'-dicycohexylcarbodiimide under ice-cooling conditions, followed by agitation at 4°C overnight and then at room temperature for 1 hour. After removal of the solvent by distillation, a saturated sodium hydrogencarbonate aqueous solution was added, followed by extraction with chloroform and drying with anhydrous magnesium sulfate. The solution was filtered, from which the solvent was distilled off and the resultant residue was subjected to silica gel column chromatography (developing solvent: dichloromethane:methanol=99:1) to obtain 130 mg of the captioned compound (yield 89%).

$^1$H-NMR(90 MHz, $CDCl_3$)δ:

1.4-2.4(m,4H), 2.55-3.5(m.3H), 2.68(s,3H). 4.35-4.9(m, 2H), 4.88(bs,2H), 6.37(bs,1H), 7.05-7.65(m,9H)

MS m/z (Pos. FAB): 526$(M+H)^+$

Example 2

2-[4-{1-(4-Bromophenylacetyl)piperidyl}]-4-(2-chlorophenyl)-9-methyl-6H-thieno-[3,2-f][1,2,4]-triazolo[4,3-a][1,4] diazepine

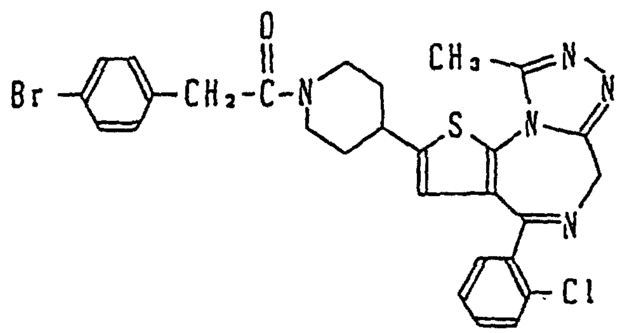

Example 1 was repeated using 4-bromophenyl acetic acid.

$^1$H-NMR(90 MHz, $CDCl_3$)δ:

1.15-2.2(m.4H), 2.49-3.3(m,5H), 2.87(s,3H), 3.55-4.1(m,1H), 4.45-5.0(m,1H), 4.88(bs,2H), 6.34(bs,1H), 6.6-7.11 (m,4H), 7.11-7.6(m,4H)

MS m/z (Pos. FAB) : 596[$(M+H)^+$, Cl=35, Br=81]

Example 3

4-(2-Chlorophenyl)-9-methyl-2-[4-[1-{4-(2-thienyl)-propionyl}piperidyl]]-6H-thieno-[3,2-f][1,2,4]-triazolo[4,3-a][1,4] diazepine

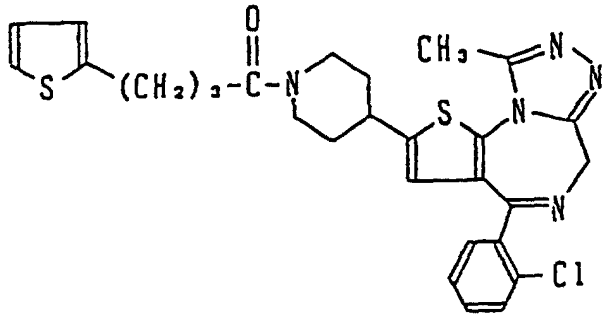

Example 1 was repeated using 4-(2-thienyl)butanoic acid.

$^1$H-NMR(90 MHz, $CDCl_3$)δ:

1.1-2.2(m,6H), 2.4-3.32(m,3H), 2.67(s,3H), 3.32-4.45(m,4H), 4.45-5.05(m,2H), 4.88(bs,2H), 6.32(bs,1H), 6.95-7.6(m,7H)

MS m/z (Pos. FAB): 550$(M+H)^+$

Example 4

4-(2-Chlorophenyl)-9-methyl-2-[4-(1-pentanoyl)-piperidyl]-6H-thieno[3,2-f][1,2,4]triazolo-[4,3-a][1,4]diazepine

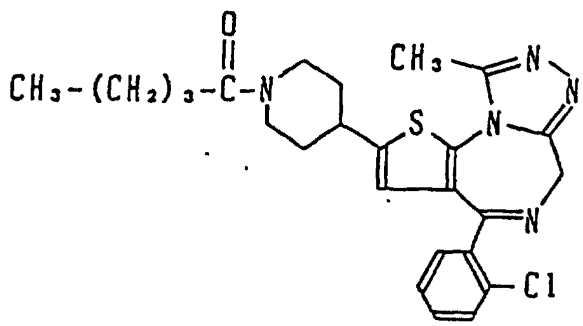

Prepared in the same manner as in Example 1 using valeric acid chloride.

$^1$H-NMR(90 MHz. $CDCl_3$)δ:

0.91(t,J=6.5Hz,3H), 1.1-2.3(m,3H), 2.31(t,J=7.2Hz,2H), 2.67(s,3H), 2.5-3.4(m,3H), 3.7-4.2(m,1H), 4.45-5.0(m, 1H), 4.88(bs,2H), 6.35(bs,2H), 7.1-7.5(m,4H)

MS m/z(Pos. FAB): 482$(M+H)^+$

## Claims

**1.** A triazolo-1,4-diazepine compound of one the formulae given below or a pharmacologically acceptable salt thereof

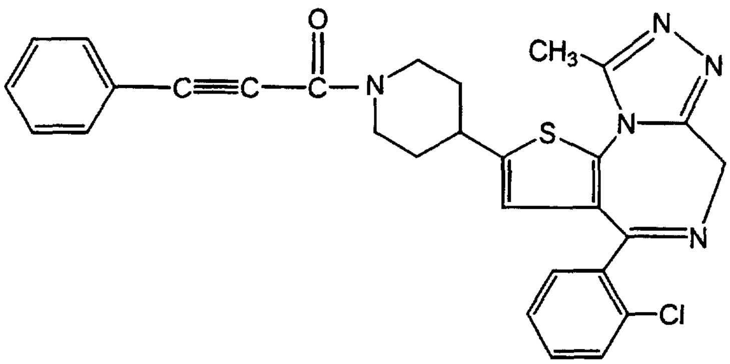

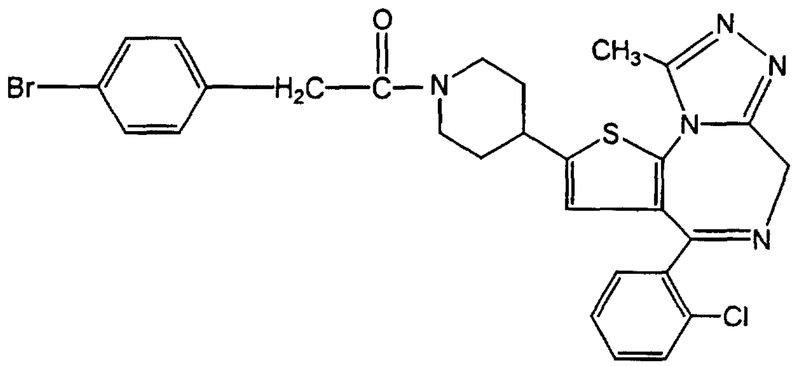

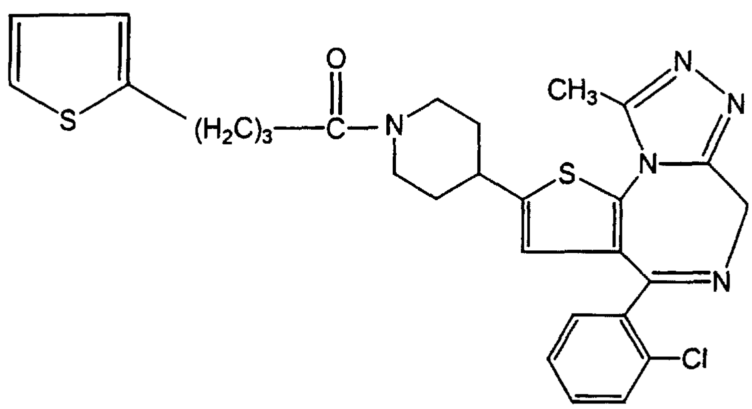

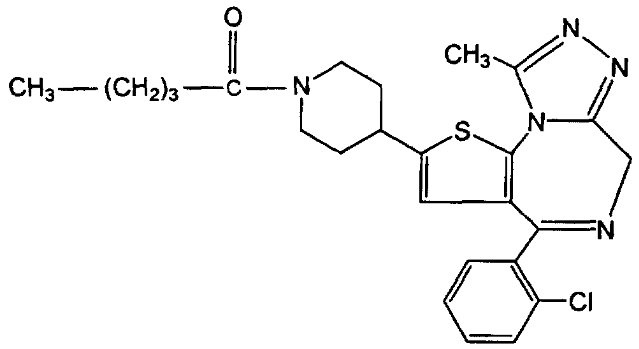

**2.** A pharmaceutical composition which comprises a pharmacologically effective amount of a compound or a salt thereof, as defined in claim 1 and a pharmacologically acceptable carrier.

**3.** The use of a compound or a salt thereof as defined in claim 1 for making a medicament for anti-PAF activity.

**4.** The use of a compound or a salt thereof as defined in claim 1 for making a medicament for the treatment or prevention of allergic disease.

**5.** The use as claimed in claim 4 in which the disease is asthma.

**Patentansprüche**

**1.** Triazolo-1,4-diazepinverbindung mit einer der nachstehend angegebenen Formeln oder ein pharmakologisch annehmbares Salz davon

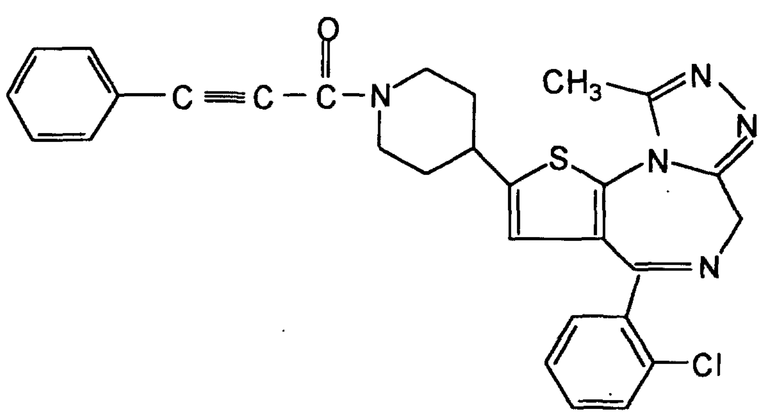

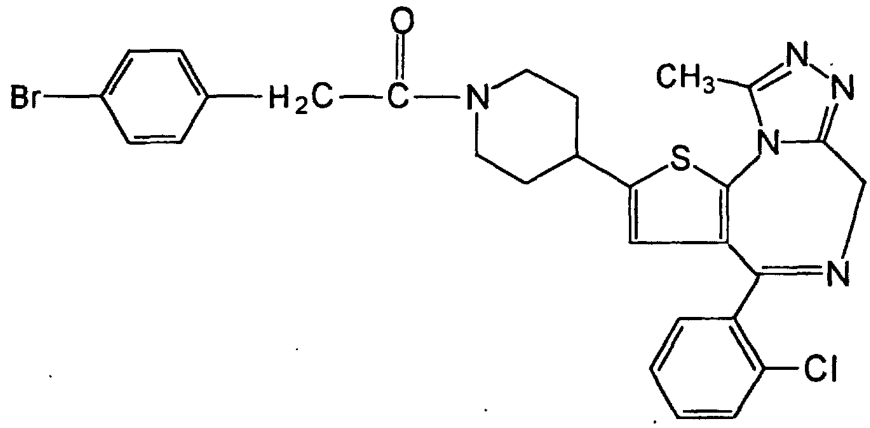

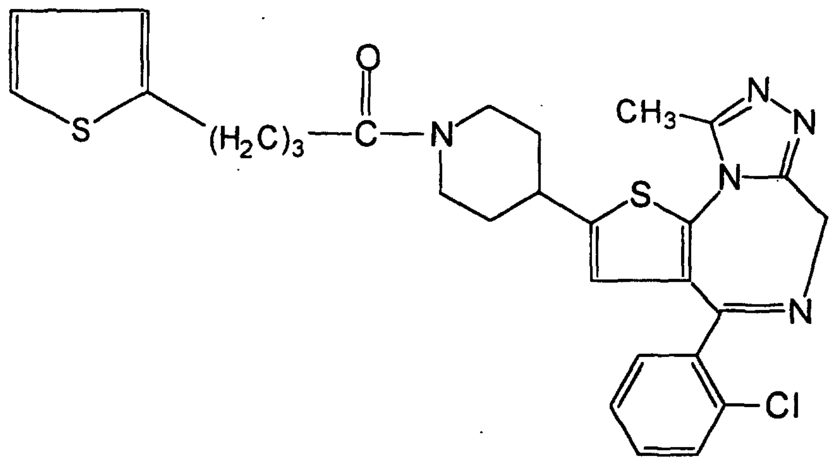

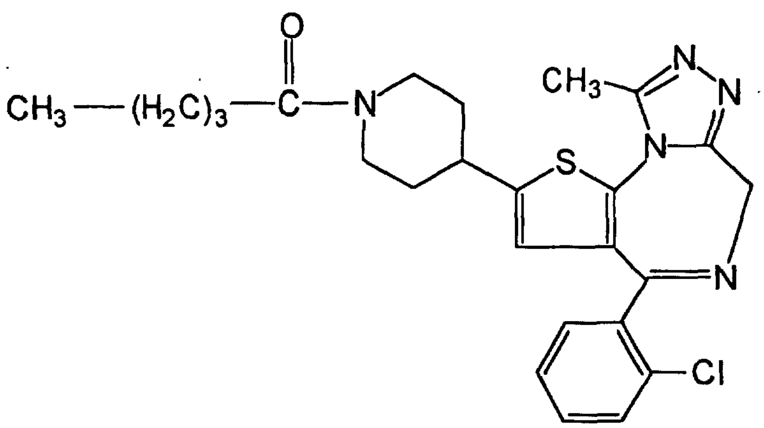

2. Pharmazeutische Zusammensetzung, die eine pharmakologisch wirksame Menge einer Verbindung oder eines Salzes davon, wie in Anspruch 1 definiert, und einen pharmakologisch annehmbaren Träger umfasst.

3. Verwendung einer Verbindung oder eines Salzes davon, wie in Anspruch 1 definiert, zum Herstellen eines Medikaments mit Anti-PAF-Aktivität.

4. Verwendung einer Verbindung oder eines Salzes davon, wie in Anspruch 1 definiert, zum Herstellen eines Medikaments zum Behandeln oder Vorbeugen einer allergischen Krankheit.

5. Verwendung gemäß Anspruch 4, worin die Krankheit Asthma ist.

## Revendications

1. Composé de triazolo-1,4-diazépine répondant à l'une quelconque des formules données ci-dessous ou sel pharmacologiquement acceptable de celui-ci,

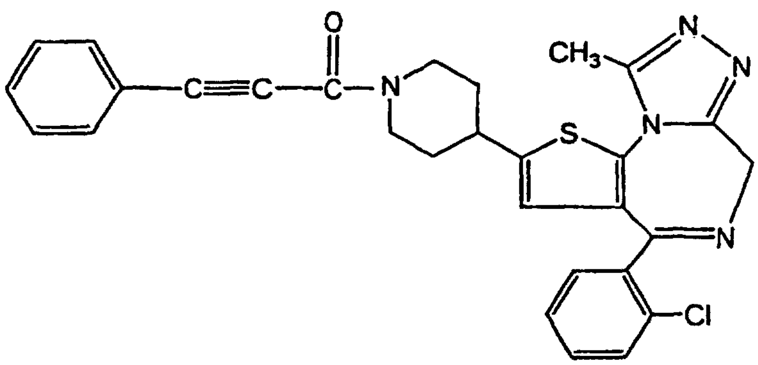

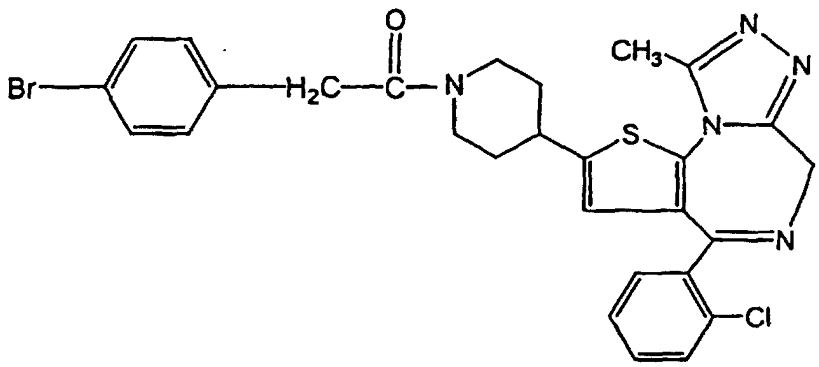

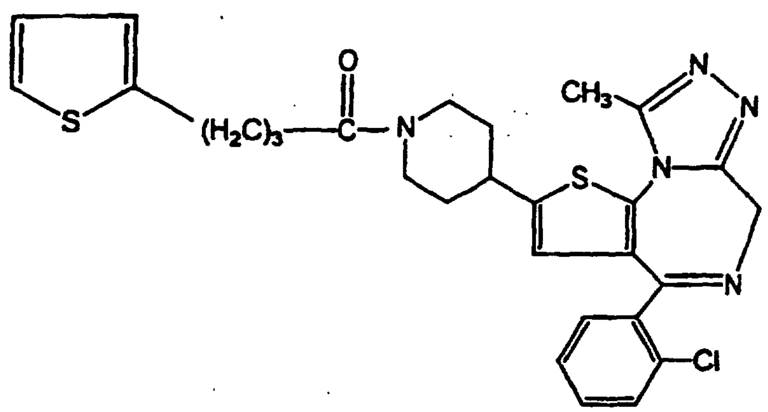

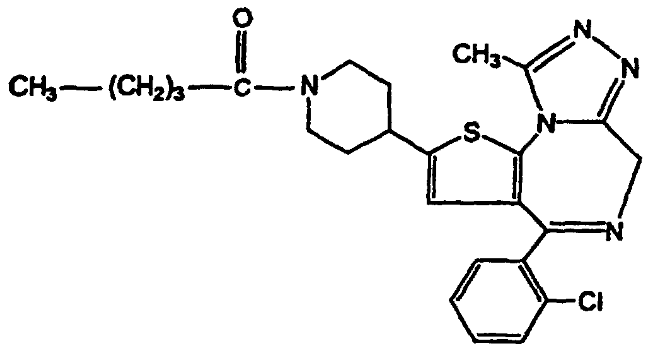

.

2. Composition pharmaceutique qui comprend une quantité pharmacologiquement efficace du composé ou du sel de celui-ci, tel que défini dans la revendication 1 et véhicule pharmacologiquement acceptable.

3. Utilisation du composé ou du sel de celui-ci tel que défini dans la revendication 1 pour fabriquer un médicament doté d'une activité anti-PAF (Facteur d'Activation des Plaquettes).

4. Utilisation du composé ou du sel de celui-ci tel que défini dans la revendication 1 pour fabriquer un médicament pour le traitement ou la prévention des maladies allergiques.

5. Utilisation telle que définie dans la revendication 4 dans laquelle la maladie est l'asthme.